# EUROPEAN PATENT APPLICATION

(11) **EP 4 789 725 A2**
(43) Date of publication of application: **12.08.2026**
(21) Application number: 26165471.9
(22) Date of filing: 14.05.2020
(51) Int. Cl.: A61P 37/00

(54) **TREATMENT OF CUTANEOUS LUPUS ERYTHEMATOSUS**

(30) Priority: 15.05.2019 EP 19174586
(62) Document number(s) of the earlier application(s) in accordance with Art. 76 EPC: 20724533.3 / 3 968 997
(71) Applicant: LEO Pharma A/S, 2750 Ballerup (DK)
(72) Inventor: NIELSEN, Jacob, 2750 Ballerup (DK); PEDERSEN, Gitte Pommergaard, 2750 Ballerup (DK); MORTENSEN, Helene, 2750 Ballerup (DK); SANDER, Camilla, 2750 Ballerup (DK); REFER, Pia Klie, 2750 Ballerup (DK)
(74) Representative: Zacco Denmark A/S

(57) **Abstract**

The present invention relates to the treatment of cutaneous lupus erythematosus. The problem to be solved by the invention is to provide a new pharmaceutical use of 3-[(3S,4R)-3-methyl-6-(7H-pyrrolo[2,3-d]pyrimidin-4-yl)-1,6-diazaspiro[3.4]octan-1-yl]-3-oxopropanenitrile. A therapeutic or preventive agent cutaneous lupus erythematosus, containing 3-[(3S,4R)-3-methyl-6-(7H-pyrrolo[2,3-d]pyrimidin-4-yl)-1,6-diazaspiro[3.4]octan-1-yl]-3-oxopropanenitrile as an active ingredient, and a pharmaceutical formulation thereof.

## Description

### FIELD OF THE INVENTION

The present invention relates to a new pharmaceutical use of Delgocitinib, 3-[(3S,4R)-3-methyl-6-(7H-pyrrolo[2,3-d]pyrimidin-4-yl)-1,6-diazaspiro[3.4]octan-1-yl]-3-oxopropanenitrile. In another aspect, the present invention relates to the treatment cutaneous lupus erythematosus. In yet another aspect, the present invention relates to a new topical pharmaceutical formulation comprising Delgocitinib.

### BACKGROUND OF THE INVENTION

Lupus erythematosus is an inflammatory autoimmune disease, which may affect only the skin (cutaneous Lupus erythematosus, CLE), but may also encompass severe systemic organ involvement (systemic Lupus erythematosus, SLE). CLE can be either acute, subacute, or chronic. Discoid lupus erythematosus (DLE) is the most common form of chronic CLE, representing 80% of all cases.

Discoid lupus erythematosus (DLE) is an autoimmune disease affecting the skin with erythematous, scaly, circumscribed lesions. Over time, these lesions can cause scarring, atrophy, dyspigmentation, and hair loss. DLE lesions generally range in size from a few mm to 15 cm in diameter and are primarily found in UV-exposed areas such as the head and neck, and to a lesser extent on the trunk and extremities. The physical appearance affects the quality of life in DLE patients and is associated with increased risk of depression and unemployment.

DLE is categorised as either localised (only lesions above the neck) or generalised (lesions both above and below the neck), of which the localised form is far more common.

There is currently no cure for DLE, and no treatments have been approved for this indication. The current standard-of-care treatments are used off-label and have limited effect. First-line treatment recommendations include sun avoidance, UV protection, and potent topical corticosteroids.

Several JAK-inhibitors are in clinical development or are already on the market. Ruxolitinib, the first FDA approved JAK1 and JAK2 inhibitor, is an oral drug which is approved in several countries/regions for treatment of patients with myelofibrosis. Tofacitinib is currently approved in the US as an oral drug for treatment of rheumatoid arthritis and is currently under development for treatment of psoriasis and atopic dermatitis.

WO2011/013785 describes nitrogen-containing spirocyclic compounds and pharmaceutical uses thereof. The compounds are stated to be JAK inhibitors useful for the prevention or treatment of e.g. autoimmune diseases, allergic diseases, psoriasis, rheumatoid arthritis and atopic dermatitis.

EP 2813228 A1 describes the pharmaceutical use of a JAK inhibitor, and more specifically a pharmaceutical composition for treating skin diseases such as senile xerosis, asteatosis, eczema and contact dermatitis.

Consequently, there is an unmet medical need for new treatment of cutaneous lupus erythematosus with high efficacy in combination with an attractive safety profile.

Currently there exists an ointment formulation, which is a paraffin-based formulation containing Delgocitinib in a suspended solid form.

Surprisingly, a stable cream formulation with Delgocitinib dissolved in the aqueos phase have been obtained.

### SUMMARY OF THE INVENTION

The present invention relates to the treatment of cutaneous lupus erythematosus comprising the compound of formula (I) 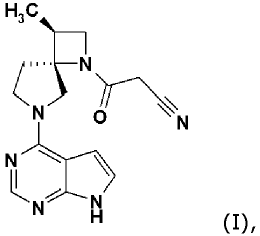 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof.

In one aspect the present invention relates to the use of the compound of formula (I) for use in the treatment of cutaneous lupus erythematosus.

In another aspect the present invention relates to the use of the compound of formula (I) for use in the treatment of discoid lupus erythematosus.

In another aspect, the present invention relates to the compound of formula (I) for use in the treatment of cutaneous lupus erythematosus. In yet another aspect, the invention relates to the compound of formula (I) for use in treatment of discoid lupus erythematosus. In yet another aspect, the invention relates to the compound of formula (I) for use in topical treatment of cutaneous lupus erythematosus, e.g. discoid lupus erythematosus. In yet another aspect, the topical formulation is a cream. In yet another aspect, the present invention relates to the use of the compound of formula (I), which is administered once daily or twice daily. In yet another aspect, the present invention relates to the use of the compound of formula (I), which is administered twice daily for 6 weeks. In another aspect, the present invention relates to the compound of formula (I) which is administered in a concentration of 20 mg/g.

In another aspect, the present invention relates to the use of the compound of formula (I) in the manufacture of a pharmaceutical composition for the treatment of cutaneous lupus erythematosus, e.g. discoid lupus erythematosus. In yet another aspect, the pharmaceutical composition is a topical formulation. In yet another aspect the topical formulation is a cream.

In another aspect, the present invention relates to a pharmaceutical composition comprising a compound of formula (I) 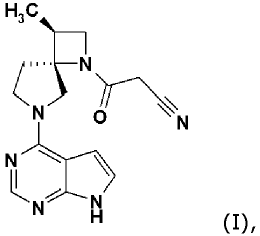 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, for the treatment of cutaneous lupus erythematosus.

In another aspect, the present invention relates to the pharmaceutical composition for the treatment of discoid lupus erythematosus.

In another aspect, the present invention relates to the pharmaceutical composition comprising the compound of formula (I) wherein the treatment is topical, e.g. treatment with a cream.

In another aspect, the present invention relates to the pharmaceutical composition wherein the compound of formula (I) is administered as 1 mg/g, 3 mg/g, 8 mg/g, and 20 mg/g concentration. In another aspect, the compound of formula (I) is administered as 20 mg/g concentration. In another aspect, the compound of formula (I) is administered as once or a twice daily application. In another aspect, the compound of formula (I) is administered as a twice daily application for 6 weeks.

In another aspect, the present invention relates to a method for treating cutaneous lupus erythematosus, e.g. discoid lupus erythematosus, in a subject in need thereof, which method comprises the step of administering to said subject a therapeutically effective amount of the compound of formula (I).

In another aspect, the present invention relates to a method for treating cutaneous lupus erythematosus, e.g. discoid lupus erythematosus, in a subject in need thereof, wherein the administration is topical.

In another aspect, the present invention relates to a method for treating cutaneous lupus erythematosus, e.g. discoid lupus erythematosus, in a subject in need thereof, wherein the topical formulation is a cream.

In another aspect, the present invention relates to a method for treating cutaneous lupus erythematosus, e.g. discoid lupus erythematosus in a subject in need thereof, wherein the compound of formula (I) is administered in a concentration of 1 mg/g, 3 mg/g, 8 mg/g, and 20 mg/g. In another aspect, the compound of formula (I) is administered in a concentration of 20 mg/g.

The present invention also provides for the use of 3-[(3S,4R)-3-methyl-6-(7H-pyrrolo[2,3-d]pyrimidin-4-yl)-1,6-diazaspiro[3,4]octan-1-yl]-3-oxopropanenitrile in the treatment of cutaneous lupus erythematosus.

The present invention further provides for the above use administered as a once daily or twice daily application.

The present invention further provides for the above use administered in a concentration of 1 mg/g, 3 mg/g, 8 mg/g, and 20 mg/g. The present invention further provides for the above use administered in a concentration of 20 mg/g.

### DETAILED DESCRIPTION OF THE INVENTION

The compound of formula (I) 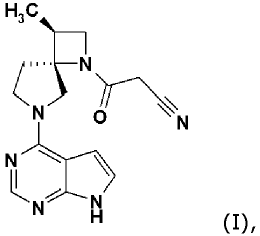 3-[(3S,4R)-3-methyl-6-(7H-pyrrolo[2,3d]pyrimidin-4-yl)-1,6-diazaspiro[3,4]octan-1-yl]-3-oxopropanenitrile, was described previously in WO2011/013785 as a JAK inhibitor for the treatment of e.g. autoimmune diseases, allergic diseases, psoriasis, rheumatoid arthritis and atopic dermatitis, and in EP 2813228 A1 for treating skin diseases such as senile xerosis, asteatosis, eczema and contact dermatitis.

The compound of formula (I) can be produced according to the method described in Preparation 6 of WO2011/013785.

The "pharmaceutically acceptable salt" may be any non-toxic salts of the compound of formula (I), and includes a salt with an inorganic acid, a salt with an organic acid, a salt with an inorganic base, a salt with an organic base, and a salt with an amino acid.

The salt with an inorganic acid includes a salt with hydrochloric acid, nitric acid, sulphuric acid, phosphoric acid, hydrobromic acid, etc. The salt with an organic acid includes salt with oxalic acid, maleic acid, citric acid, fumaric acid, lactic acid, malic acid, succinic acid, tartaric acid, acetic acid, trifluoroacetic acid, citric acid monohydrate, gluconic acid, ascorbic acid, methanesulfonic acid, benzenesulfonicacid, p-toluenesulfonic acid, etc. The salt with an inorganic base includes sodium salt, potassium salt, calcium salt, magnesium salt, ammonium salt, etc. The salt with an organic base includes a salt with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine, etc. The salt with an amino acid includes a salt with lysine, arginine, aspartic acid, glutamic acid, etc. According to well-known methods, each salt may be obtained by reacting the compound of formula (I) with an inorganic base, an organic base, an inorganic acid, an organic acid or an amino acid.

The compound of formula (I) may be labeled with isotopes, e.g. ³H, ¹⁴C, ³⁵S.

In one aspect, the compound of formula (I) or a pharmaceutically acceptable salt thereof is the substantially purified compound of formula (I) or a pharmaceutically acceptable salt thereof. In another aspect the compound of formula (I) or a pharmaceutically acceptable salt thereof is in the purity of 80% or more.

The "pharmaceutical composition" includes an oral preparation such as tablet, capsule, granule, powder, lozenge, syrup, emulsion and suspension, or a parenteral preparation such as topical agent, suppository, injection, eyedrop, nasal drug and pulmonary drug. In another aspect, the pharmaceutical composition is a topical formulation, such as ointment and cream. In yet another aspect, the pharmaceutical composition is a cream.

The pharmaceutical composition of the present invention is produced by appropriately mixing the compound of formula (I) or a pharmaceutically acceptable salt thereof with at least one type of pharmaceutically acceptable excipients or carriers in appropriate amounts according to methods known in the technical field of medicinal preparation. The content of the compound of formula (I) or a pharmaceutically acceptable salt thereof in the pharmaceutical composition depends on its dosage forms, dosage amounts, etc., and for example, is 0.1 to 100% by weight of the entire composition. For example, the content is 0.10, 0.20, 0.25, 0.30, 0.50, 0.75, 1.0, 1.25, 1.50, 1.75, 2.00, 2.25, 2.50, 2.75, 3.00, 3.25, 3.50, 3.75 or 4.00% by weight of the entire composition. In another aspect, the content of the compound of formula (I) or a pharmaceutically acceptable salt thereof in the pharmaceutical composition is 2% by weight of the entire composition.

The term "therapeutically effective amount" of the compound as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject.

The "pharmaceutically acceptable excipient" or "pharmaceutically acceptable carrier" includes various conventional organic or inorganic excipient or carrier substances for pharmaceutical materials, e.g. a disintegrant, a binder, a fluidizer, a lubricant, a solvent, a solubilizing agent, a suspending agent, a tonicity agent, a buffer, and a soothing agent for liquid preparations, and a base, an emulsifier, a surfactant, a humectant, a stabilizer, a stabilizing agent, a dispersant, a plasticizer, a pH regulator, an absorption promoter, a gelling agent, an antiseptic, a filler, a resolvent. Further, an additive including a preserving agent, a preservative, an antioxidant, and a colorant may be used, if needed.

The disintegrant includes carmellose, carmellose calcium, carmellose sodium, sodium carboxymethyl starch, croscarmellose sodium, crospovidone, low-substituted hydroxypropyl cellulose, hydroxypropyl methylcellulose, crystalline cellulose, etc.

The binder includes hydroxypropyl cellulose, hydroxypropyl methylcellulose, povidone, crystalline cellulose, white soft sugar, dextrin, starch, gelatin, carmellose sodium, gum arabic, etc.

The fluidizer includes light anhydrous silicic acid, magnesium stearate, etc.

The lubricant includes magnesium stearate, calcium stearate, talc, etc.

The solvent includes purified water, ethanol, propylene glycol, macrogol, sesame oil, corn oil, olive oil, medium chain triglyceride, etc.

The solubilizing agent includes propylene glycol, D-mannitol, benzyl benzoate, ethanol, triethanolamine, sodium carbonate, sodium citrate, etc.

The suspending agent includes benzalkonium chloride, carmellose, hydroxypropyl cellulose, propylene glycol, povidone, methyl cellulose, glyceryl monostearate, etc.

The tonicity agent includes glucose, D-sorbitol, sodium chloride, D-mannitol, etc.

The buffer or pH regulator includes phosphate or citrate salts, sodium hydrogen phosphate, sodium acetate, sodium carbonate, sodium citrate, sodium citrate dihydrate, citric acid monohydrate, hydrochloric acid, sodium hydroxide, etc.

The base includes water, animal or plant oils (e.g., olive oil, corn oil, peanut oil, sesame oil, castor oil, safflower oil, etc.), lower alcohols (e.g., ethanol, propanol, propylene glycol, 1,3-butylene glycol, phenol, etc.), higher fatty acids and esters thereof, waxes, higher alcohols, polyhydricalcohols, hydrocarbons (e.g., white soft paraffin, liquid paraffin, paraffin, hard paraffin, etc.), hydrophilic petrolatum, purified lanolin, absorptive ointment, hydrous lanolin, hydrophilic ointment, starch, pullulan, polydimethylsiloxane, isopropyl myristate, gum arabic, tragacanth gum, gelatin, dextran, cellulose derivatives (e.g., methyl cellulose, carboxymethyl cellulose, hydroxyethyl (e.g. carboxyvinyl polymer, sodium polyacrylate, polyvinyl alcohol, polyvinyl pyrrolidone, etc.), propylene glycol, macrogol (e.g., macrogol 200 to 600, etc.), and combinations of two or more kinds of these. In one aspect, the base is liquid paraffin.

The emulsifier or surfactant includes mixtures of fatty acids, especially cetyl alcohol, stearyl alcohol, and cetostearyl alcohol, macrogol cetostearyl ether, sorbitan esters, sucrose esters, ect.

The stabilizer includes sucrose esters, other sorbitan esters and poly sorbates, glyceol, propylene glycol, ethanol, cetostearyl alcohol, ect.

The acidifying agent includes strong acid selected from eg hydrochloric acid or citric acid.

The chelating agent includes ethylenediaminetetraacetic acid (EDTA), disodium edetate, ethylene glycol tetraacetic acid (EGTA), ethylene diamine, phosphoric acid, ect.

The preservative includes ethyl parahydroxybenzoate, chlorobutanol, benzyl alcohol, sodium dehydroacetate, sorbic acid, chlorocresol, dichlorobenzyl alcohol, glycerol, ethanol, propylene glycol, benzoic acid/sodium benzoate, diazolidiny urea, benzalkonium chloride, etc.

The antioxidant includes sodium sulfite, ascorbic acid, disodium edetate, trisodium edetate, alphatocopherol, butylhydroxy anisole, etc.

The colorant includes food dye, β-carotene, etc.

The pharmaceutical composition of the present invention may be administered to a mammal, such as a human being. A dosage amount depends on subjects, diseases, symptoms, dosages forms, administration routes, etc., and may be in the range from about 0.01 mg to about 1 g, e.g. from about 0.01 mg to about 600mg per day in terms of the content of the compound of formula (I) as an active ingredient. The dose may be administered at a time or in several divided doses.

A topical agent may be applied, for example, by application, inunction or spraying depending on the dosage form, etc. An application amount of the topical agent to the affected area can be selected depending on the content of the active ingredient, etc., and the topical agent can be applied, for example, at a time or in several divided amounts per day. The preferable application is once daily or twice daily.

The phrase "JAK" refers to one or more enzymes of JAK1, JAK2, JAK3, and TYK2 belonging to JAK family.

The phrase "inhibitJAK" refers to inhibiting functions of JAK to disappear or attenuate its activity, and inhibiting one or more enzymes belonging to JAK family. In one aspect, the phrase "inhibit JAK" refers to "inhibit human JAK". The inhibition of functions or the disappearance or attenuation of the activity is, in one aspect, conducted in the situations of human clinical application.

The "JAK inhibitor" may be any substances which inhibit JAK, and may be, for example, low-molecular weight compounds, nucleic acid, polypeptide, protein, antibody, vaccine, etc. In one aspect, the "JAK inhibitor" is a "human JAK inhibitor". In another aspect, the JAK inhibitor is the compound of formula (I) or a pharmaceutically acceptablesalt thereof. In yet another aspect, the JAK inhibitor is the compound of formula (I).

The term "treatment" as used herein includes amelioration of a symptom, prevention of an aggravation, maintenance of a remission, prevention of an exacerbation, and prevention of a recurrence. The term "prevention" refers to suppressing occurrence of a symptom.

The term "treatment" may also include the delaying of the progression of the disease, disorder or condition, the amelioration, alleviation or relief of symptoms and complications, and/or cure or elimination of the disease, disorder or condition.

The term "treatment" may also mean the management and care of a patient for the purpose of combating the disease, condition or disorder.

The terms "disease", "disorder" and "condition" as used herein are used interchangeably to specify a state of a patient which is not the normal physiological state of a human being.

The term "cutaneous lupus erythematosus (CLE)" may also include acute cutaneous lupus erythematosus, such as butterfly rash, photosensitivity reaction, and mucosal lupus erythematosus erosions. CLE may also include Subacute Cutaneous Lupus Erythematosus (SCLE), such as papulosquamous SCLE (also known as psoriasiform), annular variant of SCLE, and Rowell syndrome. Further, CLE may also include Chronic Cutaneous Lupus Erythematosus (CCLE), such as Discoid Lupus Erythematosus (DLE), Lupus Tumidus, Lupus Profundus, Lupus Hypertroficus et profundus, and Chilblain Lupus.

An embodiment of the present invention includes a pharmaceutical composition for treating or preventing cutaneous lupus erythematosus, e.g. discoid lupus erythematosus comprising 3-[(3S,4R)-3-methyl-6-(7H-pyrrolo[2,3-d]pyrimidin-4-yl)-1,6-diazaspiro[3.4]octan-1-yl]-3-oxopropanenitrile and a pharmaceutically acceptable excipient or carrier.

An embodiment of the present invention includes a use of 3-[(3S,4R)-3-methyl-6-(7H-pyrrolo[2,3-d]pyrimidin-4-yl)-1,6-diazaspiro[3.4]octan-1-yl]-3-oxopropanenitrile for treating or preventing cutaneous lupus erythematosus, e.g. discoid lupus erythematosus.

An embodiment of the present invention includes a method for treating or preventing cutaneous lupus erythematosus, e.g. discoid lupus erythematosus, characterized by administering to a mammal a therapeutically effective amount of 3-[(3S,4R)-3-methyl-6-(7H-pyrrolo[2,3-d]pyrimidin-4-yl)-1,6-diazaspiro[3.4]octan-1-yl]-3-oxopropanenitrile.

In one aspect the mammal is a human being.

In another aspect, the human being is a person suffering from a disease who is in need of medical care.

In another aspect, the disease is cutaneous lupus erythematosus. In another aspect, the disease is acute cutaneous lupus erythematosus, such as butterfly rash, photosensitivity reaction, and mucosal lupus erythematosus erosions. In another aspect, the disease is Subacute Cutaneous Lupus Erythematosus (SCLE), such as papulosquamous SCLE, annular variant of SCLE, and Rowell syndrome. In another aspect, the disease is Chronic Cutaneous Lupus Erythematosus, such as Discoid Lupus Erythematosus (DLE), Lupus Tumidus, Lupus Profundus, Lupus Hypertroficus et profundus, and Chilblain Lupus. In another aspect, the disease is Discoid Lupus Erythematosus (DLE).

In another aspect, the present invention relates to a therapeutic or preventive agent for cutaneous lupus erythematosus, e.g. discoid lupus erythematosus, comprising the compound of formula (I).

In another aspect, the present invention relates to a pharmaceutical composition, comprising the compound of formula (I) and one or more of a pharmaceutically acceptable excipient or carrier.

In another aspect, the present invention relates to a pharmaceutical formulation for topical administration, comprising the compound of formula (I) and one or more of a pharmaceutically acceptable excipient.

In another aspect, the present invention relates to a pharmaceutical formulation which is a cream, comprising the compound of formula (I) and one or more of a pharmaceutically acceptable excipient.

In another aspect, a pharmaceutically acceptable excipient could be one or more of a base selected from medium chain triglycerides, safflower oil, castor oil, liquid paraffin or mixtures thereof. For example the base could be liquid paraffin.

The base could be present at various amounts from about 50 mg/g to about 500 mg/g of liquid paraffin, e.g. from about 75 mg/g to about 300 mg/g, and e.g. 100 mg/g.

In another aspect, a pharmaceutically acceptable surfactant, emulsifier or stabilizer could be one or more selected from cetyl alcohol, stearyl alcohol, cetostearyl alcohol or mixtures thereof. For example the surfactant, emulsifier or stabilizer could be cetostearyl alcohol.

The surfactant, emulsifier or stabilizer could be present at various amounts from about 20 mg/g to about 100 mg/g of cetostearyl alcohol, e.g. from about 40 mg/g to about 80 mg/g, and e.g. 72 mg/g.

In another aspect, the pharmaceutically acceptable surfactant, emulsifier or stabilizer could be one or more selected from sorbitan esters, sucrose esters, macrogol cetostearyl ether or mixtures thereof. For example the surfactant or emulsifier could be macrogol cetostearyl ether.

The surfactant, emulsifier or stabilizer could be present at various amounts from about 9 mg/g to about 25 mg/g of macrogol cetostearyl ether, e.g. from about 15 mg/g to about 20 mg/g, and e.g. 18 mg/g.

In another aspect, a pharmaceutically acceptable buffer or pH regulator could be one or more of a phosphate or citrate salt, sodium acetate, sodium carbonate, sodium citrate dihydrate, hydrochloric acid or mixtures thereof. For example the buffer or pH regulator could be one or more of citric acid monohydrate and sodium citrate dihydrate.

The buffer or pH regulator could be present at various amounts from about 0.5 mg/g to about 4 mg/g of citric acid monohydrate, e.g. from about 0.7 mg/g to about 2 mg/g, and e.g. 1 mg/g.

The buffer or pH regulator could be present at various amounts from 0 mg/g to about 1 mg/g of sodium citrate dihydrate, e.g. from 0 mg/g to about 0.5 mg/g, and e.g. absent.

In another aspect, a pharmaceutically acceptable preservative could be one or more selected from benzyl alcohol, sodium dehydroacetate, sorbic acid/salts or mixtures thereof. For example the preservative could be benzyl alcohol.

The preservative could be present at various amounts from about 7 mg/g to about 13 mg/g of benzyl alcohol, e.g. from about 9 mg/g to about 11 mg/g, and e.g. 10 mg/g.

In another aspect, a pharmaceutically acceptable antioxidant could be one or more selected from sodium sulphite, disodium edetate, trisodium edetate, butylhydroxy anisole or mixtures thereof. For example the antioxidant could be butylhydroxy anisole.

The antioxidant could be present at various amounts from about 0.05 mg/g to about 0.3 mg/g of butylhydroxy anisole; e.g. from about 0.1 mg/g to about 0.25 mg/g, and e.g. 0.2 mg/g.

In another aspect, a pharmaceutically acceptable chelating agent could be one or more of EDTA, disodium edetate, EGTA, or ethylene diamine. For example the chelating agent could be disodium edetate.

The chelating agent could be present at various amounts from about 0.05 mg/g to about 1.5 mg/g of disodium edetate, e.g. from about 0.5 mg/g to about 1 mg/g, and e.g 0.6 mg/g.

In another aspect, a pharmaceutically acceptable acidifying agent could be one or more of a strong acid, e.g. hydrochloric acid or citric acid. For example the acidifying agent could be hydrochloric acid.

The acidifying agent could be present at various amounts from 0 mg/g to about 25 mg/g of hydrochloric acid, e.g. from about 10 mg/g to about 20 mg/g, and e.g. 17.7 mg/g.

In another aspect, a pharmaceutically acceptable solvent could be purified water, which could be present at various amounts from about 500 mg/g to about 900 mg/, and e.g. 760 mg/g.

In another aspect, the present invention relates to a method for preparing the present pharmaceutical formulation, wherein

The water-phase:
purified water, pH regulators , buffers, acidifying agents, preservatives, chelating agents were mixed.

The drug substance was added and dissolved in the water-phase at a temperature around 15 - 25 °C.
pH was adjusted to 4.0-4.4.

The water-phase was heated to around 65 - 75 °C.

The oil-phase:
Liquid paraffin, surfactants, emulsifiers, stabilizers, antioxidants were mixed.

The oil-phase was heated to around 65 - 75 °C.

The oil-phase was added to the water-phase and mixed.

The mixture was homogenised for approximately 20 min. and subsequently cooled to approximately 30°C. The cream was subsequently filled into the container closure.

Exemplary pharmaceutical formulations of the present invention:
Component Amount (mg/g): Delgocitinib 1; paraffin liquid 100; cetostearyl alcohol 72; macrogol cetostearyl ether 18; benzyl alcohol 10; citric acid monohydrate 0.78; butylhydroxy anisole 0.2; disodium edetate 0.6; sodium citrate dihydrate 0.31, and purified water 797.

Component Amount (mg/g): Delgocitinib 3; paraffin liquid 100; cetostearyl alcohol 72; macrogol cetostearyl ether 18; benzyl alcohol 10; citric acid monohydrate 1.0; butylhydroxy anisole 0.2; disodium edetate 0.6; 3M hydrochloric acid 1.08, and purified water 794.

Component Amount (mg/g): Delgocitinib 8; paraffin liquid 100; cetostearyl alcohol 72; macrogol cetostearyl ether 18; benzyl alcohol 10; citric acid monohydrate 1.0 butylhydroxy anisole 0.2; disodium edetate 0.6; 3M hydrochloric acid 6.43, and purified water 784.

Component Amount (mg/g): Delgocitinib 20; paraffin liquid 100; cetostearyl alcohol 72; macrogol cetostearyl ether 18; benzyl alcohol 10; citric acid monohydrate 1.0; butylhydroxy anisole 0.2; disodium edetate 0.6; 3M hydrochloric acid 17.7, and purified water 760.

In another aspect, the present invention relates to a pharmaceutical composition, comprising the compound of formula (I) and the excipients as disclosed in the investigational product (IP-1).

In another aspect, the present invention relates to a pharmaceutical formulation for topical administration comprising the compound of formula (I) and the excipients as disclosed in the investigational product (IP-2).

The present invention describes its effect in topical treatment of cutaneous lupus erythematosus. In another aspect, the present invention describes its effect in topical treatment of discoid lupus erythematosus.

The present invention provides a clinical trial to compare efficacy and safety of twice daily topical of cream application with 20 mg/g of the compound of formula (I), with a cream vehicle for 6 weeks in the treatment of adult subjects with cutaneous lupus erythematosus, more specifically discoid lupus erythematosus. All subjects will be treated with active treatment on one DLE target lesion and vehicle treatment on another DLE target lesion.

As measure of efficacy and safety the following has been defined:
The efficacy assessments is the Investigator's Global Assessment (IGA) of severity of each target lesion (score of 0 (clear) or 1 (almost clear) at week 6) and skin lesions activity and damage scores (erythema, scaling/hyperkeratosis, oedema/infiltration, dyspigmentation, scarring/atrophy (based on the Revised Cutaneous Lupus Erythematosus Disease Area and Severity Index (RCLASI)) for each target lesion. For details in IGA and RCLASI, see below.

Subject assessments of efficacy and health-related quality of life is based on Patient's Global Assessment (PaGA) of severity of each target lesion and Dermatology Life Quality Index (DLQI)

### EXAMPLES

### Clinical trial

The clinical trial is a multi-center, double-blind, randomized, vehicle controlled phase 2a trial in adult subjects with DLE with twice daily topical application of the compound of formula (I), formulated in a cream, 20 mg/g and vehicle cream for 6 weeks. Each subject must have at least 2 DLE target lesions with active disease. The 2 target lesions will be randomised 1:1 to active and vehicle treatment.

### Treatment period

At baseline (Day 1). Allocation of treatment to the 2 target lesions will be randomised (1:1) to drug cream 20 mg/g and vehicle. The treatment will be applied twice daily for 6 weeks. The first application of the investigational medicinal product (IMP) will occur at the trial site on Day 1 when all baseline assessments have been carried out. The subsequent IMP applications will be performed by the subjects at home.

During the 6-week treatment period, the subjects will return to the trial site for scheduled visits at Weeks 2, 4**,** and 6. The last application of IMP will occur in the evening before the subjects attend the visit scheduled at Week 6.

### Main inclusion criteria:

- Age 18-70 years.
- Histopathological findings (current or previous) consistent with clinical diagnosis of DLE.
- Unequivocal clinical diagnosis of 2 active DLE target lesions that are <6 months old and amenable for clinical evaluation. This includes lesions located on the scalp if they fulfil all lesion-specific eligibility criteria.
- Target lesion IGA score of at least moderate (IGA ≥3) at screening and baseline.
- A difference of ≤1 point in IGA score between the 2 target lesions at screening and baseline.
- Target lesion erythema score ≥2 at screening and baseline.

### Main exclusion criteria:

- Target lesion dyspigmentation score of 2 at screening or baseline.
- Target lesion scarring/atrophy score of 2 at screening or baseline.
- Target lesion scarring alopecia score of >0 in scalp lesions at screening or baseline.
- Medical history of SLE with clinically significant organ involvement including SLE-related pleuritis or pericarditis, and neurologic, renal and/or other major SLE-related organ system involvement. SLE joint involvement is acceptable.
- Subjects with unstable or significant SLE disease activity based on the experience of the investigator.
- Other skin conditions at screening or baseline that would interfere with the evaluation of DLE.
- Immunosuppressive/immunomodulating therapy, e.g. methotrexate, cyclosporine, azathioprine, retinoids, dapsone within 4 weeks prior to baseline.
- Systemic prednisolone >7.5 mg/day or changed dose within 4 weeks prior to baseline (nasal and inhaled corticosteroids are allowed).
- Treatment with the following medications:
   - Oral antimalarial treatment with hydroxychloroquine >6.5mg/kg body weight/ day, or chloroquine > 4mg/kg body weight/day, or changed dose within 12 weeks prior to baseline.
   - Quinacrine combined with either hydroxychloroquine or chloroquine within 12 weeks prior to baseline.
   - Drugs known to interact with antimalarials (e.g. digoxin, cimetidine) within 12 weeks prior to baseline.
- Treatment with topical corticosteroids, calcineurin inhibitors, and phosphor-diesterase-4 (PDE-4) inhibitors within 2 weeks prior to baseline.
- Use of systemic antibiotics or cutaneously applied antibiotics on the target lesions within 2 weeks prior to baseline.
- UV therapy within 2 weeks prior to baseline.
- Any procedure impairing the skin barrier (e.g. incision) within 2 cm from the border of any of the target lesions within 4 weeks prior to baseline.
- Receipt of live (attenuated) vaccines within 4 weeks prior to baseline.
- Products containing Hypericum perforatum (St John's Wort) within 4 weeks prior to baseline.
- Treatment with any marketed biological therapy or investigational biologic agents:
   - Any cell-depleting agents including but not limited to rituximab: within 6 months prior to baseline, or until lymphocyte count returns to normal, whichever is longer.
   - Other biologics: within 3 months or 5 half-lives, whichever is longer, prior to baseline.
- History of any active skin infection within 1 week prior to baseline.
- Clinically significant infection within 4 weeks prior to baseline which, in the opinion of the investigator, may compromise the safety of the subject in the trial.
- Tuberculosis requiring treatment within 12 months prior to screening and/or subjects with a positive blood test for tuberculosis at screening.

### Investigator's Global Assessment (IGA) for discoid lupus erythematosus (DLE)

The IGA is used to rate the severity of the subject's global disease and is based on a 5 point scale ranging from 0 (clear) to 4 (severe). The investigator will use the IGA as a lesion-specific assessment to rate the severity of DLE for each of the 2 target lesions separately.

Investigator's Global Assessment (IGA) for discoid lupus erythematosus (DLE)

| Score | Disease severity | Morphological description |
|---|---|---|
| 0 | Clear | No activity signs of DLE (no erythema, no scaling/hyperkeratosis, no oedema/infiltration). Damage signs may be present (dyspigmentation and/or scarring/atrophy). |
| 1 | Almost clear | Pink or faint erythema only. No scaling/hyperkeratosis, no oedema/infiltration. |
| 2 | Mild | Pink or faint erythema. Slight scaling (mostly fine scales) and/or slight dermal oedema. |
| 3 | Moderate | Prominent erythema. Slight scaling (mostly fine to circumscribed adherent scales) and/or slight to definite dermal oedema. |
| 4 | Severe | Prominent red, dark red, purple, or violaceous colour. Crusting may be present. Severe scaling with follicular plugging and/or dermal oedema with skin induration. |

### Revised Cutaneous Lupus Erythematosus Disease Area and Severity Index (RCLASI)

The RCLASI is a validated scoring system to assess disease activity and damage in patients with CLE, taking both anatomical region and morphological aspects into account.

The RCLASI skin lesions activity and damage signs scores are applied to the target lesions only. The individual sign scores and sum will give a quantitative assessment of the treatment effect. A high RCLASI score indicates more severe disease. In addition, a full RCLASI assessment will be done at baseline to describe the overall CLE disease burden in the trial population.

The total skin disease activity score is defined as the sum of scores for erythema, scaling/hyperkeratosis, and oedema/infiltration:
Erythema: 0=Absent; 1=Pink, faint; 2=Red; 3=Dark red, purple/violaceous/ crusted/haemorrhagic.
Scaling/hyperkeratosis: 0=Absent; 1=Circumscribed adherent scaling/follicular plugging; 2 = Verrucous hyperkeratosis.
Oedema/infiltration: 0=Absent; 1=Slight, just palpable; 2=Palpable and visible.

The total skin disease damage score is defined as the sum of scores for dyspigmentation and scarring/atrophy:
Dyspigmentation: 0=Absent; 1a=Hypopigmentation; 1b=Hyperpigmentation; 2=Hypo- and hyperpigmentation.
Scarring/atrophy: 0=Absent; 1=Initial scarring; 2=Severe firm/atrophic/vermicular scarring.

### Patient's Global Assessment of disease severity (PaGA)

PaGA is a self-assessment of the subjects' perception of disease severity.

Subjects will assess the disease severity of each target lesion using the PaGA. The assessment will be made using the 5-point scale:
0 = Clear; 1 = Almost clear; 2 = Mild; 3 = Moderate; 4 = Severe.

### Dermatology Life Quality Index (DLQI)

DLQI is a validated questionnaire with content specific to those with dermatology conditions. It consists of 10 items addressing the subject's perception of the impact of their skin disease on different aspects of their quality of life over the last week such as dermatology-related symptoms and feelings, daily activities, leisure, work or school, personal relationships, and the treatment. Each item is scored on a 4-point Likert scale (0 = "not at all /not relevant" 1 = "a little"; 2 = "a lot" 3 = "very much"). The total score is the sum of the 10 items (0 to 30); a high score is indicative of a poor quality of life.

### Investigational product (IP-1)

| Ingredient | Drug product (mg/g) | Vehicle (mg/g) |
|---|---|---|
| **Drug substance** | | |
| the compound of formula (I) | 20 | - |

| **Excipients** | | |
|---|---|---|
| Paraffin liquid | 100 | 100 |
| Cetostearyl alcohol | 72 | 72 |
| Macrogol cetostearyl ether | 18 | 18 |
| Benzyl alcohol | 10 | 10 |
| Citric acid monohydrate | 1 | 0.59 |
| Sodium citrate | 1 | 0.57 |
| Disodium edetate | 1.3 | 1.3 |
| Hydrochloric acid | 4.99 | - |
| Butylhydroxy anisole | 0.2 | 0.2 |
| Purified water | up to 1 g | up to 1 g |

### Investigational product (IP-2)

| Ingredient | Drug product (mg/g) | Vehicle (mg/g) |
|---|---|---|
| **Drug substance** | | |
| the compound of formula (I) | 20 | - |

| **Excipients** | | |
|---|---|---|
| Paraffin liquid | 100 | 100 |
| Cetostearyl alcohol | 72 | 72 |
| Macrogol cetostearyl ether | 18 | 18 |
| Benzyl alcohol | 10 | 10 |
| Citric acid monohydrate | 1 | 0.59 |
| Sodium citrate dihydrate | - | 0.57 |
| Disodium edetate | 0.6 | 0.6 |
| 3M Hydrochloric acid | 17.7 | - |
| Butylhydroxy anisole | 0.2 | 0.2 |
| Purified water | up to 1 g | up to 1 g |

### CLAUSES

In view of the description, the present invention has in particular provided:
1. A compound of general formula (I) 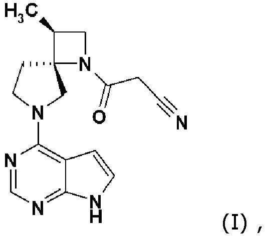 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof for use in the treatment of cutaneous lupus erythematosus.
2. The compound for use according to clause 1 wherein the treatment is for discoid lupus erythematosus.
3. The compound for use according to clause 1 or 2 wherein the treatment is topical treatment.
4. The compound for use according to any one of the preceding clauses wherein the compound of formula (I) or a pharmaceutically acceptable salt thereof is administered in a cream.
5. The compound for use according to any one of the preceding clauses wherein the compound of formula (I), or a pharmaceutically acceptable salt thereof is administered in a concentration of 20 mg/g.
6. The compound for use according to any one of the preceding clauses wherein the compound of formula (I), or a pharmaceutically acceptable salt thereof is administered as a twice daily application.
7. A pharmaceutical composition for use in the treatment of cutaneous lupus erythematosus comprising a compound of formula (I) 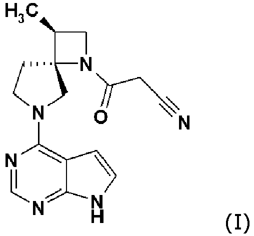 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, as an active ingredient.
8. The pharmaceutical composition for use according to clause 7, wherein the treatment is discoid lupus erythematosus.
9. The pharmaceutical composition for use in the treatment according to clause 7 or 8 wherein the pharmaceutical composition is topical.
10. The pharmaceutical composition for use in the treatment according to any one of the clauses 7-9 wherein the pharmaceutical composition is a cream.
11. The pharmaceutical composition for use in the treatment according to any one of the clauses 7-10 wherein the compound of formula (I), or a pharmaceutically acceptable salt thereof is administered in a concentration of 20 mg/g.
12. The pharmaceutical composition for use in the treatment of according to any one of the clauses 7-11 wherein the compound of formula (I), or a pharmaceutically acceptable salt thereof is administered as a twice daily application.
13. A method for use in the treatment of cutaneous lupus erythematosus in a subject in need thereof, which method comprises the step of administering to said subject a therapeutically effective amount of the compound of formula (I) 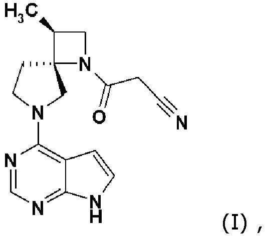 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof.
14. The method for use according to clause 13 wherein the treatment is discoid lupus erythematosus.
15. The method for use in the treatment according to clause 13 or 14 wherein the administration is topical.
16. The method for use in the treatment according to any one of the preceding clauses 13-15 wherein the topical administration is a cream.
17. The method for use in the treatment according to any one of the preceding clauses 13-16 wherein the compound of formula (I), or a pharmaceutically acceptable salt thereof is administered in a concentration of 20 mg/g.
18. A therapeutic or preventive agent for cutaneous lupus erythematosus, comprising a compound of formula (I) 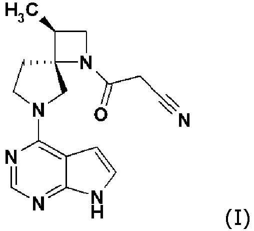 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, as an active ingredient.
19. The therapeutic or preventive agent according to clause 18 for the treatment or prevention discoid lupus erythematosus.
20. The therapeutic or preventive agent according to clause 18 or 19 wherein the therapeutic or preventive agent for discoid lupus erythematosus ,is a topical agent.
21. The therapeutic or preventive agent according to any one of the preceding clauses 18-20 wherein the therapeutic or preventive agent is a cream.
22. The therapeutic or preventive agent according to any one of the preceding clauses 18-21 wherein the compound of formula (I), or a pharmaceutically acceptable salt thereof, is administered in a concentration of 20 mg/g.
23. The therapeutic or preventive agent according to any one of the preceding clauses 18-22 wherein the compound of formula (I), or a pharmaceutically acceptable salt thereof, is administered as a twice daily application.
24. A pharmaceutical formulation for topical administration comprising
   - the compound of formula (I) and,
      one or more of a pharmaceutically acceptable excipient selected from:
   - a base, such as liquid paraffin,
   - surfactants, emulsifiers, stabilizers, such as cetostearyl alcohol and macrogol cetostearyl ether,
   - pH regulators, buffers, such as phosphate or citrate salts and hydrochloric acid,
   - preservatives,
   - antioxidants,
   - chelating agents,
   - acidifying agents, and
   - purified water.
25. The pharmaceutical formulation according to clause 24, wherein the base is liquid paraffin, which is present in an amount from about 50 mg/g to about 500 mg/g.
26. The pharmaceutical formulation according to clause 25, wherein the liquid paraffin is present in an amount from about 75 mg/g to about 300 mg/g, and especially 100 mg/g.
27. The pharmaceutical formulation according to any one of the clauses 24-26, wherein the surfactant, emulsifier, stabilizer, are one or more of cetostearyl alcohol and macrogol cetostearyl ether.
28. The pharmaceutical formulation according to clause 27, wherein the cetostearyl alcohol is present in an amount from about 20 mg/g to about 100 mg/g.
29. The pharmaceutical formulation according to clause 28, wherein the cetostearyl alcohol is present in an amount from about 40 mg/g to about 80 mg/g, and especially 72 mg/g.
30. The pharmaceutical formulation according to clause 27, wherein the macrogol cetostearyl ether is present in an amount from about 9 mg/g to about 25 mg/g.
31. The pharmaceutical formulation according to clause 30, wherein the macrogol cetostearyl ether is present in an amount from about 15mg/g to about 20 mg/g, and especially 18 mg/g.
32. The pharmaceutical formulation according to any one of the preceding clauses 24-31, wherein the buffer, pH regulator is selected from phosphate or citrate salts.
33. The pharmaceutical formulation according to clause 32, wherein the buffer. pH regulator citric acid monohydrate, which is present in an amount of from about 0.5 mg/g to about 4 mg/g.
34. The pharmaceutical formulation according to clause 33, wherein the citric acid monohydrate is present in an amount from about 0.7 mg/g to about 2 mg/g, and especially 1 mg/g.
35. The pharmaceutical formulation according to clause 32, wherein the buffer, pH regulator is sodium citrate dihydrate, which is present in an amount of from 0 mg/g to about 1 mg/g.
36. The pharmaceutical formulation according to clause 35, wherein the sodium citrate dihydrate is absent.
37. The pharmaceutical formulation according to any one of the clauses 24-36, wherein the preservative is benzyl alcohol.
38. The pharmaceutical formulation according to clause 37, wherein the benzyl alcohol is present in an amount from about 7 mg/g to about 13 mg/g.
39. The pharmaceutical formulation according to clause 38, wherein the benzyl alcohol is present in an amount from about 9 mg/g to about 11 mg/g, and especially 10 mg/g.
40. The pharmaceutical formulation according to any one of the clauses 24-39, wherein the antioxidant is butylhydroxy anisole.
41. The pharmaceutical formulation according to clause 40, wherein the butylhydroxy anisole is present in an amount of from about 0.05 mg/g to about 0.3 mg/g.
42. The pharmaceutical formulation according to clause 41, wherein the butylhydroxy anisole is present in an amount of from about 0.1 mg/g to about 0.25 mg/g, and especially 0.2 mg/g.
43. The pharmaceutical formulation according to any one of the clauses 24-42, wherein the chelating agent is EDTA.
44. The pharmaceutical formulation according to clause 43, wherein the chelating agent is disodium edetate.
45. The pharmaceutical formulation according to clause 44, wherein the disodium edetate is present in an amount from about 0.05 mg/g to about 1.5 mg/g.
46. The pharmaceutical formulation according to clause 45, wherein the disodium edetate is present in an amount from 0.5 mg/g to about 1 mg/g, and especially 0.6 mg/g.
47. The pharmaceutical formulation according to any one of the clauses 24-46, wherein the acidifying agent is hydrochloric acid .
48. The pharmaceutical formulation according to clause 47, wherein the hydrochloric acid is present in an amount from 0 mg/g to about 25 mg/g.
49. The pharmaceutical formulation according to clause 48, wherein the hydrochloric acid is present in an amount from about 10 mg/g to about 20 mg/g, and especially 17.7 mg/g.
50. The pharmaceutical formulation according to any one of the clauses 24-49, wherein the purified water is present in an amount from about 500 mg/g to about 900 mg/g.
51. The pharmaceutical formulation according to clauses 50, wherein the purified water is present in an amount of 760 mg/g.
52. The pharmaceutical formulation according to any one of the clauses 24-51, wherein the compound of formula (I) is present in an amount of 1 mg/g, 3 mg/g, 8 mg/g, or 20 mg/g.
53. The pharmaceutical formulation for topical administration comprising
   the compound of formula (I), 1 mg/g,
   paraffin liquid, 100 mg/g,
   cetostearyl alcohol, 72 mg/g,
   macrogol cetostearyl ether, 18 mg/g,
   benzyl alcohol, 10 mg/g,
   citric acid monohydrate, 0.78 mg/g,
   butylhydroxy anisole, 0.2 mg/g,
   disodium edetate, 0.6 mg/g,
   sodium citrate dihydrate, 0.31 mg/g, and
   purified water, 797 mg/g.
54. The pharmaceutical formulation for topical administration comprising
   the compound of formula (I), 3 mg/g,
   paraffin liquid, 100 mg/g,
   cetostearyl alcohol, 72 mg/g,
   macrogol cetostearyl ether, 18 mg/g,
   benzyl alcohol, 10 mg/g,
   citric acid monohydrate, 1.0 mg/g,
   butylhydroxy anisole, 0.2 mg/g,
   disodium edetate, 0.6 mg/g,
   3M hydrochloric acid, 1.08 mg/g, and
   purified water, 794 mg/g.
55. The pharmaceutical formulation for topical administration comprising the compound of formula (I), 8 mg/g, paraffin liquid, 100 mg/g,
   cetostearyl alcohol, 72 mg/g,
   macrogol cetostearyl ether 18 mg/g,
   benzyl alcohol, 10 mg/g,
   citric acid monohydrate, 1.0 mg/g,
   butylhydroxy anisole, 0.2 mg/g,
   disodium edetate, 0.6 mg/g,
   3M hydrochloric acid, 6.43 mg/g, and
   purified water, 784 mg/g,
56. The pharmaceutical formulation for topical administration comprising
   the compound of formula (I), 20 mg/g,
   liquid paraffin, 100 mg/g,
   cetostearyl alcohol, 72 mg/g,
   macrogol cetostearyl ether, 18 mg/g,
   benzyl alcohol, 10 mg/g,
   citric acid monohydrate, 1 mg/g,
   butylhydroxy anisole, 0.2 mg/g,
   disodium edetate, 0.6 mg/g,
   3M hydrochloric acid, 17.7 mg/g, and
   purified water, 760 mg/g.
57. The pharmaceutical formulation according to any one of the clauses 24-56 wherein the formulation is a cream.

## Claims

1. A compound of general formula (I) 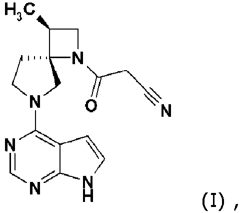 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof for use in the treatment of cutaneous lupus erythematosus.

2. The compound for use according to claim 1 wherein the treatment is for discoid lupus erythematosus.

3. The compound for use according to any one of the preceding claims wherein the compound of formula (I), or a pharmaceutically acceptable salt thereof is administered in a concentration of 20 mg/g.

4. A pharmaceutical composition for use in the treatment of cutaneous lupus erythematosus comprising a compound of formula (I) 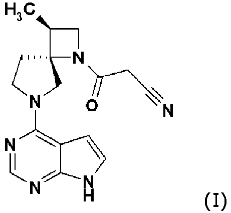 3-[(3S,4R)-3-methyl-6-(7H-pyrrolo[2,3d]pyrimidin-4-yl)-1,6-diazaspiro[3,4]octan-1-yl]-3-oxopropanenitrile, or a pharmaceutically acceptable salt thereof, as an active ingredient.

5. The pharmaceutical composition for use according to claim 4, wherein the treatment is discoid lupus erythematosus.

6. The pharmaceutical composition for use in the treatment according to any one of the preceding claims 4-5 wherein the compound of formula (I), or a pharmaceutically acceptable salt thereof is administered in a concentration of 20 mg/g.

7. A pharmaceutical formulation for topical administration comprising
- the compound of formula (I) and,
one or more of a pharmaceutically acceptable excipient selected from:
- a base, such as liquid paraffin,
- surfactants, emulsifiers, stabilizers, such as cetostearyl alcohol and macrogol cetostearyl ether,
- pH regulators, buffers, such as phosphate or citrate salts, hydrochloric acid,
- preservatives,
- antioxidants,
- chelating agents,
- acidifying agents, and
- purified water.

8. The pharmaceutical formulation according to claim 7, wherein the base is liquid paraffin in an amount from about 50 mg/g to about 500 mg/g.

9. The pharmaceutical formulation according to claim 7 or 8, wherein cetostearyl alcohol is present in an amount from about 20 mg/g to about 100 mg/g.

10. The pharmaceutical formulation according to any one of the claims 7-9, wherein macrogol cetostearyl ether is present in an amount from about 9 mg/g to about 25 mg/g.

11. The pharmaceutical formulation according to any one of the claims 7-10, wherein the phosphate or citrate salt is citric acid monohydrate in an amount of from about 0.5 mg/g to about 4 mg/g.

12. The pharmaceutical formulation according to one of the claims 7-10, wherein the phosphate or citrate salt is sodium citrate dihydrate in an amount of from 0 mg/g to about 1 mg/g.

13. The pharmaceutical formulation according to any one of the claims 7-12, wherein the preservative is benzyl alcohol in an amount from about 7 mg/g to about 13 mg/g.

14. The pharmaceutical formulation according to any one of the claims 7-13, wherein the antioxidant is butylhydroxy anisole is in an amount of from about 0.05 mg/g to about 0.3 mg/g.

15. The pharmaceutical formulation according to any one of the claims 7-14, wherein the chelating agent is disodium edetate in an amount from about 0.05 mg/g to about 1.5 mg/g.

16. The pharmaceutical formulation according to any one of the claims 7-15, wherein the acidifying agent is hydrochloric acid in an amount from 0 mg/g to about 25 mg/g.

17. The pharmaceutical formulation according to any one of the claims 7-16 comprising the compound of formula (I), 20 mg/g; liquid paraffin, 100 mg/g; cetostearyl alcohol, 72 mg/g; macrogol cetostearyl ether, 18 mg/g; benzyl alcohol, 10 mg/g; citric acid monohydrate, 1 mg/g; butylhydroxy anisole, 0.2 mg/g; disodium edetate, 0.6 mg/g; 3M hydrochloric acid, 17.7 mg/g; and purified water, 760 mg/g.
